# EUROPEAN PATENT SPECIFICATION

(11) **EP 4 070 156 B1**
(45) Date of publication and mention of the grant of the patent: **12.11.2025**
(21) Application number: 20816265.1
(22) Date of filing: 04.12.2020
(51) Int. Cl.: G02C 1/00, G02C 5/12, A61B 3/04, G02C 13/00

(54) **METHOD AND SYSTEM FOR DETERMINING A PRESCRIPTION FOR AN EYE OF A PERSON**
VERFAHREN UND SYSTEM ZUR BESTIMMUNG EINES REZEPTS FÜR EIN AUGE EINER PERSON
PROCÉDÉ ET SYSTÈME POUR DÉTERMINER UNE PRESCRIPTION POUR L' IL D'UNE PERSONNE

(30) Priority: 06.12.2019 EP 19306596; 10.07.2020 FR 2007340
(43) Date of publication of application: 12.10.2022
(73) Proprietor: Essilor International, 94220 Charenton-Le-Pont (FR)
(72) Inventor: LE GALL, Mathieu, 75009 PARIS (FR); FREZAL, Thibaut, 75009 PARIS (FR); FRARIER, Damien, 75009 Paris (FR); PERRIN, Jean-Luc, 94220 CHARENTON-LE-PONT (FR); BONNIN, Thierry, 94220 CHARENTON-LE-PONT (FR); JOUARD, Ludovic, 94220 CHARENTON-LE-PONT (FR); ERNOULT, Lucie, 94220 CHARENTON-LE-PONT (FR)
(74) Representative: Cabinet Novitech
(86) International application number: PCT/EP2020/084758
(87) International publication number: WO 2021/110985

(56) References cited:
- EP-A1- 1 157 656
- EP-A1- 3 521 904
- WO-A2-2018/178493
- IT-A1- UA20 163 978
- KR-A- 20090 100 508
- US-A- 4 561 739
- US-A- 5 488 439
- US-A1- 2003 174 282
- US-A1- 2011 047 807
- US-A1- 2015 338 678
- JANG JONG BAL: "test glasses for multifocus lens", 20 March 2008 (2008-03-20), XP055693355, Retrieved from the Internet <URL:http://translation.com> [retrieved on 20200508]

## Description

### FIELD OF THE INVENTION

The invention relates to an eyewear. The invention further relates to a method for providing an eyeglass adapted to a user and to a method for comparing a first lens and a second lens using such eyewear.

### BACKGROUND OF THE INVENTION

Ophthalmic lenses intended to be held in a frame usually involve a prescription. The ophthalmic prescription can include a positive or negative power prescription as well as an astigmatism prescription. These prescriptions correspond to corrections enabling the wearer of the lenses to correct defects of his vision. A lens is fitted in the frame in accordance with the prescription and with the position of the wearer's eyes relative to the frame.

Lenses suitable for presbyopic wearers are multifocal lenses or progressive addition lenses, wherein the value of the power correction is different for far vision and near vision, due to the difficulties of accommodation in near vision. For example progressive addition lenses are generally determined by optimization, based on a certain number of constraints imposed on the different features of the lens.

Nevertheless, some wearer may have difficulties to adapt to the use of progressive addition lenses despite normal binocular vision and other normal clinical findings.

It is not usual for an eye care practitioner to be able to demonstrate different type of lenses. A few demonstrators exists proposing to switch the lens from a demonstrator headwear to present different type of lens, or different type of optical designs for multifocal lenses. However to this end, each trial lens is placed independently in front of an eye of the wearer and thus the fitting position is to be done each time one change lens set/type.

In addition, the comparison between different lenses in-store needs quick switching between lenses.

KR 2009 0100508 relates to a prior art test glasses for multifocal lenses and test methods therefore.

Thus, there is a need for clients to experiment and compare different lenses benefits by quickly and efficiently switching between the lenses.

One object of the present invention is to provide a demonstration tool to support lens sales in-store allowing to switch together lenses while maintaining client's fittings parameters for another try.

### SUMMARY OF THE INVENTION

To this end, the invention proposes an eyewear according to claim 1.

Advantageously, such eyewear allows quickly switching two lens holders while maintaining the fitting's parameters of a client thus allowing an efficient switch between the lenses to be tested, in particular when the frame headwear is worn by the wearer.

Indeed, thanks to the eyewear, the pupillary distance and the fitting height adjustments are set once for all the tested lens holders.

Thus, such eyewear ensures the client to efficiently experiment and compare different lenses benefits.

The eyewear satisfies rapidity requirement and less eye care practitioner's settings and manipulation on the client for the test.

The invention discloses two main embodiments:
- the eyewear is worn alone and used as an eyeglass frame.
- the eyewear is worn over spectacles used by the user.

Advantageously, the second embodiment, besides taking advantage of the features of the first embodiment, allows to determine the compatibility of the lenses of the eyewear and the frame of the spectacles used by the user.

Indeed, thanks to the second embodiment of the invention, one can determine if the spectacles worn by the user need to be adapted or replaced to fit with the lenses of the eyewear.

According to further embodiments which can be considered alone or in combination:
- each lens of the pair of ophthalmic lenses is movable relative to the associated lens holder along an axis corresponding to a nasal-temporal axis of the user when the eyewear is worn by the user such that the associated pair of ophthalmic lenses is positioned in front of the eyes of the user ;
- the or each lens holder is magnetically fastened to the frame headwear;
- the lens holder comprises two circles jointed by a bridge, the lenses being positioned within the circles ;
- a position of a nosepad of the frame headwear relative to the frame headwear is adjustable vertically when the eyewear is worn by the user;
- a position of the lens holder relative to the frame headwear is adjustable vertically when the eyewear is worn by the user such that the associated pair of ophthalmic lenses is positioned in front of the eyes of the user;
- the eyewear comprises a set of at least two lens holders, each pair of ophthalmic lenses being different from one lens holder to another of the set, preferentially having different optical powers and/or different optical designs from one lens holder to another;
- the single vision lenses of the first subset have a first optical power and the progressive addition lenses of the second subset have an addition of same dioptric value as the first optical power ;
- the set of at least one lens holder comprises:
   ∘a first subset of at least one first lens holder, each first lens holder having an associated pair of single vision lenses having at least a first optical power, and
   ∘ a second subset of at least one second lens holder, each second lens holder having an associated pair of bifocal lenses or trifocal lenses having at least one optical power equal to the first optical power;
- the set of at least one lens holder comprises:
   ∘ a first subset comprising at least a first lens holder having an associated pair of single vision lenses having a first optical power, and a second lens holder having an associated pair of single vision lenses having a second optical power distinct from the first optical power, and
   ∘ a second subset comprising at least a third lens holder, the third lens holder having an associated pair of third lenses, the third lenses comprising at least a near vision zone having an optical power equal to the first optical power and a far vision zone having an optical power equal to the second optical power, the third lenses being progressive additional lenses or bifocal lenses or trifocal lenses;
- the lenses have different optical designs and/or different optical powers and/or different tint.
- the lens holder comprises at least two landmarks each of which locates a reference point of the ophthalmic lens;
- the reference point corresponds to the optical center of the ophthalmic lens or to the far vision reference point of the ophthalmic lens ;
- at least part of the landmarks are located on the peripherical parts of the ophthalmic lens ;
- each lens holder comprises a lens rim in which the associated ophthalmic lens is fix and mounted to said frame headwear via said lens rim, and wherein at least one of the landmarks is located on the lens rim ;
- at least part of the landmarks are configured to indicate the vertical position of the reference point of the ophthalmic lens ;
- at least part of the landmarks are configured to indicate the horizontal position of the reference point of the ophthalmic lens.
- at least one lens of the lens holder comprises a vertical graduated scale to evaluate the fitting height of the lens.
- at least one lens of the lens holder comprises a landmark to indicate the position of a near vision zone.

Another object of the invention is directed to a method according to claim 13.

According to further embodiments of the method which can be considered alone or in combination:
- the method further comprises moving vertically the position of the lens holder relative to the frame headwear or vertically a position of a nosepad of the frame headwear relative to the frame headwear to align a pupil center of an eye of the user with a landmark of a lens placed in front of the eye;
- the eyewear comprises a set of at least two lens holders and the method further comprises:
   ∘ providing another lens holder of the set;
   ∘ switching the lens holder with the other lens holder;
   ∘ for each lens of the other lens holder, moving the position of the lens relative to the other lens holder to adjust the position of the lens of the other lens holder to the pupillary distance of the user;
   ∘ each lens holder comprises at least two landmarks each of which locates a reference point of each ophthalmic lens and the method further comprises: providing the frame headwear with least two lens holder mounted to the frame headwear in a predetermined mounted position;
   ∘ moving the position of the ophthalmic lenses associated to the lens holders relative to the frame headwear to adjust the position of the lens to the position of the pupil of the user, for example the pupillary distance, using the landmarks to locate the reference point on the ophthalmic lens.
- when the user wear spectacles, the method further comprises:
   ∘ mounting the frame headwear over the spectacles, and
   ∘ switching in and/or out the lens holder over the spectacles.

Another object of the disclosure is directed to a method for comparing two different lenses as defined in claim 13, the method comprising at least:
- providing a frame headwear;
- providing a first lens holder and a second lens holder,
- for each lens holder of the first lens holder and a second lens holder:
   ∘ fastening the lens holder to the frame headwear;
   ∘ for each lens of the lens holder, moving the position of the lens relative to the lens holder to adjust the position of the lens to the pupillary distance of the user,
- switching from the first lens holder to the second lens holder at least once after adjusting the position of the lens to the pupillary distance of the user.

According to further embodiments of the method which can be considered alone or in combination:
- the method further comprises moving vertically the position of the lens holder relative to the frame headwear or vertically a position of a nosepad of the frame headwear relative to the frame headwear to align a pupil center of an eye of the user with the landmarks ;
- the method further comprises moving horizontally the position of the lens holder relative to the frame headwear to align a pupil center of an eye of the user with the landmarks ;
- the eyewear comprises a set of at least two lens holders, and at least one frame headwear comprising fixations for fastening the lens holders, or at least two frames, each fastened immovably to a lens holder, the method further comprising:
   ∘ providing another lens holder of the set;
   ∘ switching the lens holder with the other lens holder;
   ∘ for each lens of the other lens holder, moving the position of the lens relative to the other lens holder to adjust the position of the lens of the other lens holder to the pupillary distance of the user using the landmarks to locate the reference point on each ophthalmic lens;
- when the user wear spectacles, the method further comprises:
   ∘ mounting the frame headwear over the spectacles,
   ∘ switching in and/or out the lens holder over the spectacles,
   ∘ evaluating the fitting height of the lens using the vertical graduated scale of the lens,
   ∘ determining if the landmark indicating a near vision zone of the lens of the lens holder is in front of any part of the corresponding lens of the spectacles.

Another object of the disclosure is directed to a method for comparing two different lenses, the method comprising at least:
- providing a first and a second eyewear, each eyewear comprising a frame headwear configured to be worn by a user, at least one lens holder mounted to the frame headwear in a predetermined mounted position and each lens holder comprising an associated ophthalmic lens attached to said lens holder, each lens and lens holder being configured to be movable relative to the frame headwear while being mounted to said frame headwear, in each lens holder comprises at least two landmarks each of which locates a reference point of each ophthalmic lens,
- for each eyewear, moving the position of the ophthalmic lens relative to frame headwear to adjust the position of the lens to the position of the pupil of the user, for example the pupillary distance, using the landmarks to locate the reference point on each ophthalmic lens,
- switching from the first eyewear to the second eyewear at least once after adjusting the position of the lens to the pupillary distance of the user.

According to an embodiment, the lenses have different optical designs and/or different optical powers and/or different tint.

### BRIEF DESCRIPTION OF THE DRAWINGS

Embodiments of the invention will now be described, by way of example only, and with reference to the following drawings in which:
- Figures 1 and 2 are schematic representations of an eyewear according to an embodiment of the invention; in figure 1, the eyewear is assembled contrary to figure 2;
- Figure 3 is a schematic representation of a lens holder of the eyewear illustrated on figures 1 and 2;
- Figure 4 is an illustration of a part of the lens holder of figure 3 comprising a right lens;
- Figures 5 is a schematic representation of an eyewear according to an embodiment of the invention;
- Figure 6 is a schematic representation of a lens holder of the eyewear illustrated on figures 5;
- Figures 7 and 8 illustrates respectively a wrong and a right setting of the fitting height of the eyewear with respect to the wearer's eyes;
- Figure 9 is a schematic representation of a lens holder of the eyewear worn over spectacles according to an embodiment of the invention;
- Figure 10 is an illustration of a chart-flow of a method for providing an eyeglass adapted to a user using an eyewear according to the invention; and
- Figure 11 is an illustration of a chart-flow of a method or comparing a first lens and a second lens according to an embodiment of the invention.

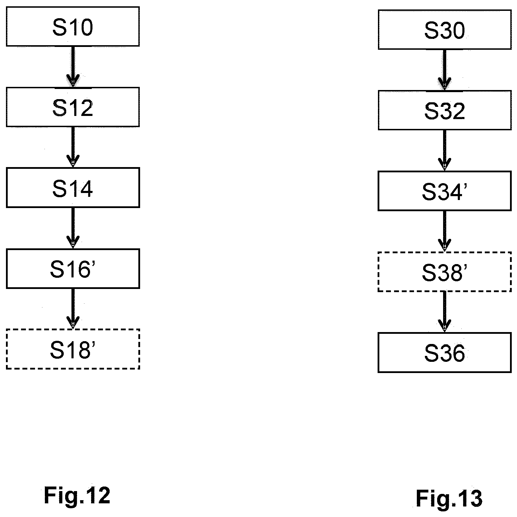

- Figure 12 is an illustration of a chart-flow of a method for providing an eyeglass adapted to a user using an eyewear according to an embodiment of the invention as illustrated on figure 5 and 6; and
- Figure 13 is an illustration of a chart-flow of a method or comparing a first lens and a second lens according to an embodiment of the invention as illustrated on figure 5 and 6.

Elements in the figures are illustrated for simplicity and clarity and have not necessarily been drawn to scale. For example, the dimensions of some of the elements in the figure may be exaggerated relative to other elements to help improve the understanding of the embodiments of the present invention.

### DETAILED DESCRIPTION OF PREFERRED EMBODIMENTS

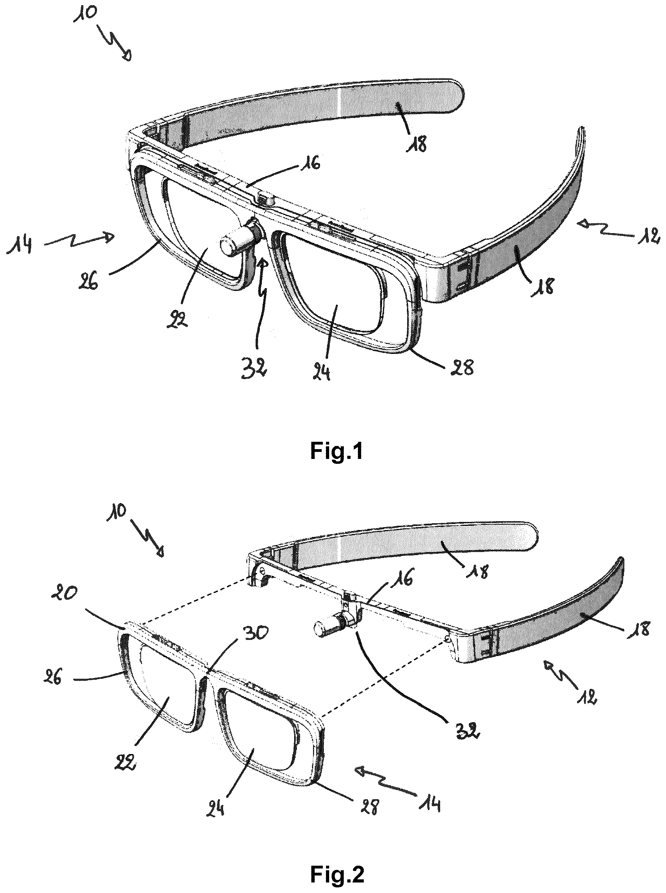

The invention relates to an eyewear comprising a frame headwear and at least one lens holder. Figures 1 and 2 represent an example of eyewear 10 comprising a frame headwear 12 and a lens holder 14. In figure 1, the eyewear is assembled contrary to figure 2 wherein the lens holder 14 is not yet fastened to the frame headwear 12.

The frame headwear 12 is configured to be worn by a user. To this end, the frame headwear 12 comprises a front part 16, a first and second temples 18. The first and second temples 18 are configured to be attached to the front part 16, for example using hinges and screws. The frame headwear is defined as being part of a frame that doesn't bear any lenses while the lens holder is not fastened to it.

Each lens holder 14 is configured to be removably fastened to the frame headwear 12 in a predetermined mounted position.

For example, the lens holder may be advantageously magnetically fastened to the frame headwear allowing a quick, repeatable and reliable positioning of the lens holder relative to the frame headwear 12. Two lens holders can thus be easily switched with repeatable replacement on the frame headwear.

The frame headwear fastened with the lens holder may be used as an eyeglass frame by the user or may be positioned by the wearer over spectacles already worn by the user. To this end, the frame headwear is configured to be mounted over possible spectacles of the user.

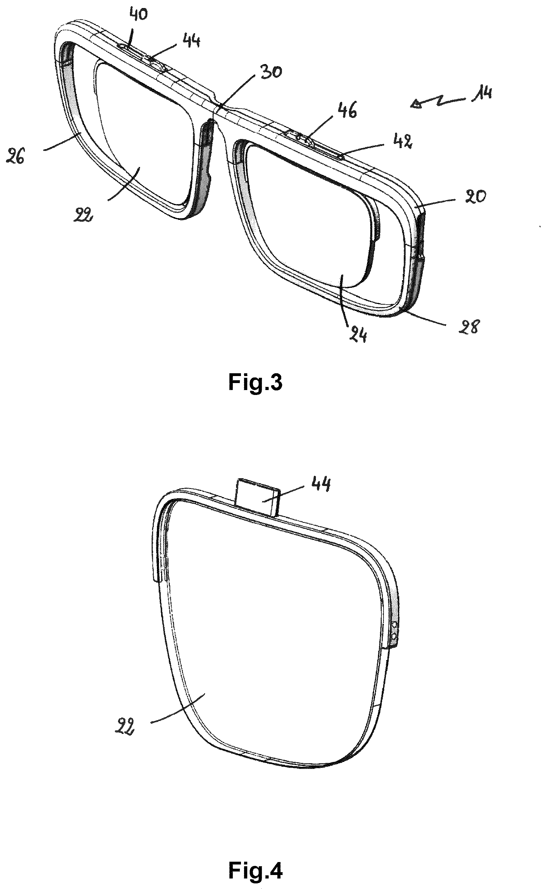

The frame headwear 12 further comprises a nose pad 32 arranged to rest on the nose of the user when the eyewear 10 in worn by the user. With reference to figure 3, the lens holder 14 comprises a frame 20 and an associated pair of optical lenses attached to said lens holder 14 via the frame 20. The associated pair of optical lenses comprises a first optical lens 22 and a second optical lens 24. The first and second optical lenses 22, 24 are preferably ophthalmic lenses. The first optical lens 22 and a second optical lens 24 are coupled to a lens holder ensuring an efficient switching between two lens holders in particular when the frame headwear is worn by the wearer.

Advantageously, the frame 20 of the lens holder 14 comprises a first circle 26 and a second circle 28 linked by a bridge 30 allowing to handle the lens holder quickly without putting fingers on the lenses when switching two lens holders. Indeed, this enables to preserve the lens from being moved relative to the lens holder during handling and from being soiled during the quick switching, which would be detrimental to the quality of the comparison. Each of the first and second circles 26, 28 comprises frame rims arranged to receive respectively the first and second lenses 22, 24 such that the lenses 22, 24 are positioned within a corresponding circle 26, 28.

The first lens 22 and the second lens 24 are arranged in order to be placed in front of respectively a right eye and a left eye (not illustrated on figures 1 to 3) of the user when the lens holder 14 is attached to the frame headwear 12 and the eyewear 10 in worn by the user.

The first lens 22 and the second lens 24 are each configured to be movable independently of one another relative to the lens holder 14 while being attached to said lens holder 14. Advantageously, each lens 22, 24 of the pair is movable relative to the lens holder along an axis corresponding to a nasal-temporal axis of the user when the eyewear is worn by the user such that the associated pair of ophthalmic lenses is positioned in front of the eyes of the user.

In other words, the lens holder comprises means for adjusting the position of each lens 22, 24 along an axis corresponding to a nasal-temporal axis of the user when the eyewear is worn allowing to adjust the pupillary distance independently for each eye. For example, a first slide 40 and a second slide 42 are arranged in the frame 20 of the lens holder and each first lens 22 and second lens 24 comprises respectively a first complementary mean 44 and a second complementary mean 46, as illustrated on figure 4. The first complementary mean 44 is arranged and configured to be inserted in the first slide 40 such that when an eye care practitioner slides the first complementary mean 44 in the first slide 40, the first lens 22 moves relative to the lens holder along an axis corresponding to the nasal-temporal axis of the user allowing the setting of the pupillary distance for the right eye.

In the same manner, the second complementary mean 46 is arranged and configured to be inserted in the second slide 42 such that when the eye care practitioner slides the second complementary mean 46 in the second slide 42, the first lens 22 moves relative to the lens holder along an axis corresponding to the nasal-temporal axis of the user allowing the setting of the pupillary distance for the left eye independently of the setting of the pupillary distance for the right eye.

Advantageously, a position of the frame headwear 12 is further adjustable vertically relative to the user when the eyewear is worn by the user such that the associated pair of ophthalmic lenses is well positioned in front of the eyes of the user while the lens holder 14 is attached to the frame headwear 12.

In other words, the frame headwear comprises means for adjusting the position of the nosepad 32 of the frame headwear 12 relative to the frame headwear 12 along a vertical axis when the eyewear is worn by the user allowing the setting of the fitting height. Thanks to the invention, the fitting height is set once for a lens holder and is then maintained for other lens holders.

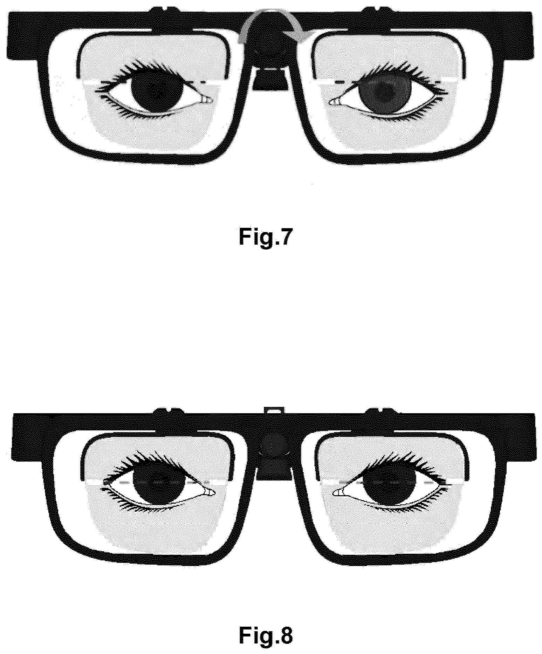

For example, the lenses 22, 24 comprises landmarks and the frame headwear 14 comprises a system for moving vertically the position of the frame headwear 12 relative to the user allowing the alignment of the pupil centers of the eyes of the user with the landmarks of the lenses 22, 24 as illustrated on figures 7 and 8. Figure 7 illustrates that the fitting height of the eyewear is misaligned with respect to the wearer's eyes while in figure 8, the setting of the fitting height is correct.

For example, the system for moving vertically the position of the nosepad 32 of the frame headwear 12 relative to the frame headwear 12 when the eyewear is worn may comprise a rack and a pinion configured to cooperate with the rack.

According to an embodiment, a position of the lens holder 14 relative to the frame headwear 12 may further be adjustable vertically when the eyewear is worn by the user such that the associated pair of ophthalmic lenses is positioned in front of the eyes of the user.

According to a preferred embodiment, the eyewear 10 comprises a set of a plurality of lens holders. Each lens holder of the set is configured to be removably fastened to the frame headwear in the same manner and as described hereinbefore and each lens of the associated pair of ophthalmic lenses is also movable relative to the lens holder as described hereinbefore.

Each pair of ophthalmic lenses of the set comprises a right lens 22 and a left lens 24 intended to be respectively placed in front of the right eye and the left eye of the user when wearing the eyewear.

The right lenses of the set have same right contour and optical centers of the right lenses are positioned in same place relative to the right contour. In the same manner, the left lenses of the set have same left contour and optical centers of the left lenses are positioned in same place relative to the left contour

Each pair of ophthalmic lenses is different from one lens holder to another of the set allowing the comparison of the benefits of the lens holders of the set. In other words, at least one among the right lens and the left lens of a lens holder is different from one lens holder to another of the set.

According to a preferred embodiment, the pairs of ophthalmic lenses of the set have different optical powers and/or different optical designs from one lens holder to another.

In the sense of the invention an "optical design" designates the set of parameters that allows defining the surfaces of an ophthalmic lens. For example, it may comprise the surface equation, position and orientation of a surface of a face of an ophthalmic lens, such equation, position and orientation being defined in a coordinate system.

According to a first embodiment, the set of lens holders comprises a first subset of at least one first lens holder, and a second subset of at least one second lens holder.

Each first lens holder has an associated pair of single vision lenses and each second lens holder has an associated pair of progressive addition lenses.

Preferably, at least one single vision lens of the first subset has a first optical power and at least one progressive addition lens of the second subset has at least one zone having the first power or an addition of same dioptric value as the first optical power.

Advantageously, such eyewear with such set of lens holders according to the first embodiment allows the client to efficiently experiment and compare progressive addition lens benefits relative to those of the single vision lens by quickly switching the corresponding lens holders while maintaining the fitting's parameters of the client in particular when the frame headwear is worn by the user.

According to a second embodiment, each first lens holder of the first subset has an associated pair of single vision lenses and each second lens holder of the second subset has an associated pair of bifocal lenses or trifocal lenses.

Preferably, at least one single vision lens of the first subset has a first optical power and at least one bifocal or trifocal lens of the second subset has at least one zone having the first power.

Advantageously, such eyewear with such set of lens holders according to the second embodiment allows the client to efficiently experiment and compare bifocal or trifocal lens benefits relative to those of the single vision lens by quickly switching the corresponding lens holders while maintaining the fitting's parameters of the client in particular when the frame headwear is worn by the user.

According to a third embodiment, the set of at least one lens holder comprises a first subset comprising at least a first lens holder and a second lens holder and a second subset comprising at least a third lens holder.

The first lens holder comprises an associated pair of single vision lenses having a first optical power P1, and the second lens holder comprises an associated pair of single vision lenses having a second optical power P2 distinct from the first optical power P1.

Preferably, at least one third lens holder comprises an associated pair of third lenses comprising at least a near vision zone having an optical power equal to the first optical power P1 and a far vision zone having an optical power equal to the second optical power P2.

Of course, the third lenses may be progressive additional lenses, bifocal lenses or trifocal lenses.

Advantageously, such eyewear with such set of lens holders according to the third embodiment allows the client to efficiently experiment and compare benefits of different lenses: progressive addition lenses, bifocal lenses, trifocal lenses and single vision lenses, by quickly switching the corresponding lens holders while maintaining the fitting's parameters of the client in particular when the frame headwear is worn by the client.

Of course all these embodiments are compatible and the lenses may have different optical designs and/or different optical powers and/or different tint and/or different functional coatings allowing an easily comparison of their benefits by a client. A set may comprise different subsets for single vision lenses, progressive addition lenses, bifocal lenses and/or trifocal lenses. For example, a set of lens holders may comprise:
- a first subset of first lens holders, each first lens holder having an associated pair of single vision lenses with a first optical power, the first optical power may be different from one single vision lens to another,
- a second subset of second lens holders, each second lens holder having an associated pair of progressive addition lenses having a near vision zone with a near vision optical power and/or a far vision zone with a far vision optical power such that at least one of the near vision optical power and the far vision power is equal to at least one first optical power of single vision lenses of a first lens holder, and/or
- a third subset of third lens holders, each third lens holder having an associated pair of bifocal or trifocal lenses having a near vision zone with a near vision optical power and/or a far vision zone with a far vision optical power such that at least one of the near vision optical power and the far vision power is equal to at least one first optical power of single vision lenses of a first lens holder.

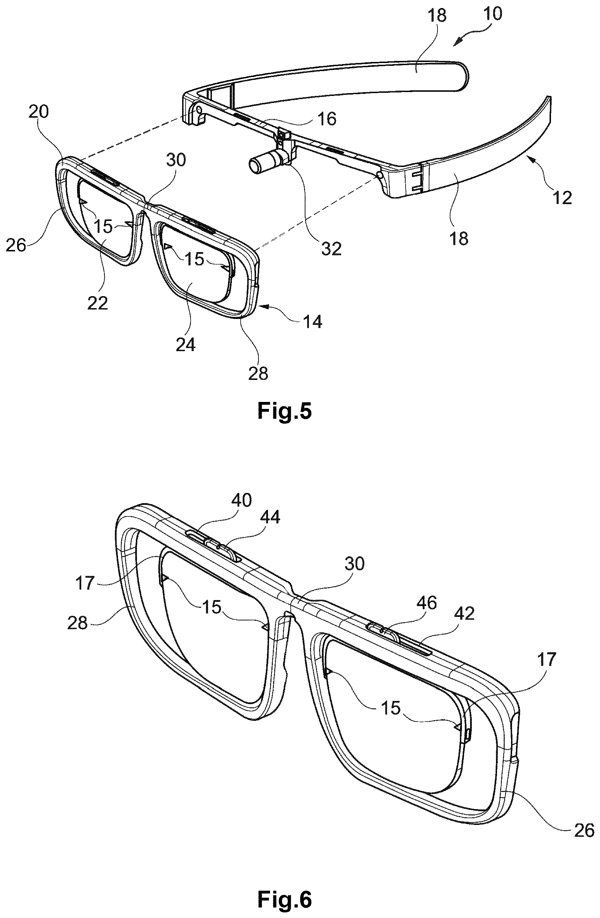

According different embodiments of the disclosure, the lens holder may comprise at least two landmarks 15 represented in figure 5 and 6, each of the at least two landmarks 15 locates a reference point of the ophthalmic lens.

The reference point of the ophthalmic lens may correspond to the optical center of the ophthalmic lens or to the far vision reference point of the ophthalmic lens or to the near vision reference point of the ophthalmic lens.

The landmarks 15 may be used by the eye care practitioner to align the reference point with the pupil of the user when gazing in a given direction or at a given distance.

For example, while the user gazes at far distance, for example at a visual target at a distance greater than 4 meters, the eye care practitioner may align the landmarks locating the far vision reference point of the ophthalmic lens with the center of the pupil of the user.

Advantageously, the landmarks make it much easier for the eye care practitioner to check the position of the ophthalmic lenses and if necessary, to accurately position the ophthalmic lenses before the eyes of the user.

According to an embodiment of the disclosure, at least part, for example all, of the landmarks may be located directly on the ophthalmic lens itself, preferably on the peripherical parts of the ophthalmic lenses so as to reduce visual discomfort for the user.

According to an embodiment of the disclosure, the ophthalmic lens may comprise a landmark, like a square or any shape defining a contour, that may refer the optical center of the lens. For example, the geometrical center of the landmark of the ophthalmic lens corresponds to the optical center of the ophthalmic lens. Thus, by aligning the pupils of the user with the geometrical center of the landmark, one aligns the pupils of the user with the optical center of each of the lenses.

According to an embodiment of the disclosure, at least part of the landmarks is configured to indicate the horizontal position of the ophthalmic lens.

Advantageously, the adjustment of the positions of the first and second ophthalmic lens along the nasal-temporal axis is facilitated by the landmarks indicating the horizontal position of the reference point.

According to an embodiment of the disclosure, at least part, for example all, of the landmarks are configured to indicate the vertical position of the reference point of the ophthalmic lens. Advantageously, the adjustment of the fitting height is facilitated by the landmarks indicating the vertical position of the reference point.

For example, the ophthalmic lenses 22, 24 may comprises landmarks and the frame headwear 14 comprises a system for moving vertically the position of the ophthalmic lenses relative to the user allowing the alignment of the pupil centers of the eyes of the user with the landmarks of the lenses 22, 24 as illustrated on figures 7 and 8. Figure 7 illustrates that the fitting height of the eyewear is misaligned with respect to the user's eyes while in figure 8, the setting of the fitting height is correct.

Each lens holder comprises landmarks each of which locates a reference point of the lens, allowing an easy and accurate positioning of the lenses before the eyes of the user.

The lenses 22, 24 may comprise landmarks and the frame headwear 14 may comprise a system for moving vertically the position of the frame headwear 12 relative to the user allowing the alignment of the pupil centers of the eyes of the user with the landmarks of the lenses 22, 24 as illustrated on figures 7 and 8. Figure 7 illustrates that the fitting height of the eyewear is misaligned with respect to the wearer's eyes while in figure 8, the setting of the fitting height is correct.

The eyewear 10 may comprise a set of a plurality of pair of ophthalmic lenses. Each lens of the plurality of pair of ophthalmic lenses may be associated to at least one landmark so as to facilitate the accurate positioning to each ophthalmic lens before the eye of the user.

According to an embodiment of the disclosure, the lenses 22, 24 may comprise a vertical graduated scale 50 to evaluate the fitting height of the lens.

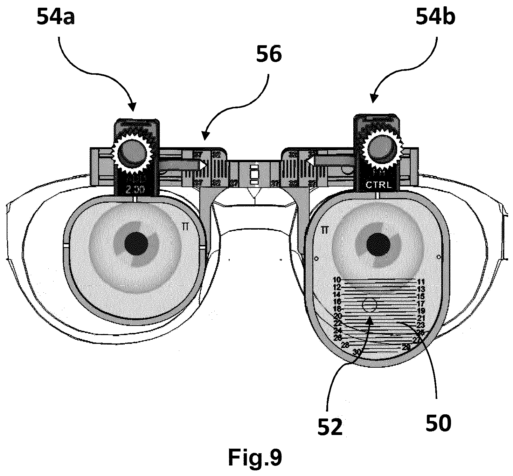

Advantageously, when the eyewear is worn over spectacles worn by the user, when the pupils and the optical centers of the lenses 22, 24 are aligned, the distance between the optical center of the lens and the bottom of the frame of spectacles worn by the user can be evaluated using the vertical graduated scale 50 as illustrated on figure 9.

According to an embodiment of the disclosure, the lenses 24, 24 may comprise a landmark 52 to indicate the position of a near vision zone.

Advantageously, when the pupils and the optical centers of the lenses 22, 24 are aligned, the compatibility between the lens 22,24 and the spectacles worn by the user can be determined. Indeed, if the landmark 52 indicating a near vision zone in the lenses 22,24

is not in front of any area in the corresponding lens of the spectacles, the said spectacles should be adapted or replaced to fit with the lenses 22, 24.

According to an embodiment of the disclosure, the lens holder 14 may comprise two scroll wheels 54, the first scroll wheel 54a allowing to adjust the distance between the two lenses of the lens holder 14, the second scroll wheel 54b allowing to adjust the vertical height of the lenses relative to the lens holder 14.

The lens holder 14 may comprise a graduated scale 56 allowing to measure directly the Inter Pupillary Distance (IPD) when the pupils and the optical centers of the lenses are aligned.

Another object of the invention relates to a method for providing an eyeglass adapted to a user using an eyewear according to the invention and as described hereinbefore.

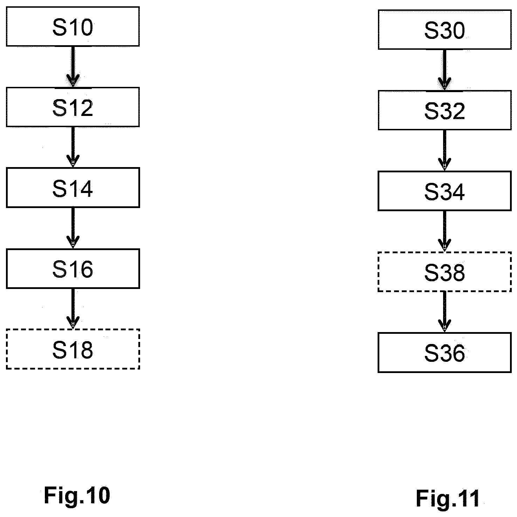

With reference to figure 10, the method comprises at least:
- providing S10 the frame headwear;
- providing S12 a lens holder;
- fastening S14 the lens holder to the frame headwear;
- for each first and second lenses of the lens holder when the eyewear is worn by the user, moving S16 the position of the lens relative to the lens holder to adjust the position of each lens to the pupillary distance of the eyes of the user.

Advantageously, the method further comprises moving S18 vertically the position of the nosepad of the frame headwear relative to the frame headwear and/or moving vertically the position of the lens holder relative to the frame headwear to align a pupil center of an eye of the user with a landmark of a lens placed in front of the eye allowing the setting of the fitting height of the eyewear to the user.

According to the invention, the eyewear comprises a set of lens holders as disclosed hereinbefore. Each lens holder of the set comprises a pair of optical lenses. Thus, the method further comprises:
- providing another lens holder of the set;
- switching the lens holder with the other lens holder;
- for each lens of the other lens holder, moving the position of the lens relative to the other lens holder to adjust the position of the lens of the other lens holder to the pupillary distance of the user.

Thanks to the invention, the fitting height is set once for a lens holder and is then maintained for other lens holders.

In addition, the eyewear allows the switching of both lenses at the same time and the switching between various lenses is very quick and thus efficient for a successful experience and comparison between the presented lenses in particular when the frame headwear is worn by the wearer.

According to another embodiment compatible with the previous ones, the method further comprises, for each first and second lenses of the lens holder when the eyewear is worn by the user, moving S16' the position of the ophthalmic lens associated to the lens holder relative to the frame headwear to adjust the position of the lens to the position of the pupil of the user, for example the pupillary distance, using the landmarks to locate the reference point on the ophthalmic lens.

Advantageously, the method may further comprise moving S18' vertically the position of the lens holder relative to the frame headwear or vertically a position of a nosepad of the frame headwear relative to the frame headwear to align a pupil center of an eye of the user with the landmarks.

According to another embodiment compatible with the previous ones, when the user already wears spectacles, the method further comprises mounting the frame headwear over the spectacles, and switching the lens holder on the spectacles by unfastening the lens holder from the frame headwear allowing an efficient comparison between the user's spectacles alone and the user's spectacles with the presented lenses. For example, if the user wears unifocal lenses having a correction in power and cylinder, the presented lenses can have an additional function that may be optical (addition for example) or complementary nature (a treatment, a hue, polarization...).

Advantageously, the method may further comprise:
- evaluating the fitting height of the lens using the vertical graduated scale of the lens,
- determining if the landmark indicating a near vision zone of the lens of the lens holder is in front of any part of the corresponding lens of the spectacle.

Another object of the disclosure relates to a method for comparing a first lens and a second lens, for example adapted to be placed in front of a same eye. The method comprises at least:
- providing S30 a frame headwear;
- providing S32 a first lens holder and a second lens holder. The first lens holder comprises at least the first lens arranged to be placed in front of the corresponding eye when the eyewear is worn. The second lens holder comprises at least the second lens arranged to be placed in front of the same eye when the eyewear is worn.
- for each lens holder of the first lens holder and the second lens holder:
   ∘ fastening the lens holder to the frame headwear;
   ∘ for each lens of the lens holder, moving S34 the position of the lens relative to the lens holder to adjust the position of the lens to the pupillary distance of the eye of the user,
- switching S36 from the first lens holder to the second lens holder at least once after adjusting the position of the lens to the pupillary distance of the user in particular when the frame headwear is worn by the wearer.

According to another embodiment compatible with the previous ones, the method may further comprise, for each lens of the lens holder, moving S34' the position of the lens relative to the lens holder to adjust the position of the lens to the pupillary distance of the eye of the user using the landmarks.

Preferably the method further comprises moving S38 vertically the position of the frame headwear relative to the user to align a pupil center of an eye of the user with a landmark of a lens placed in front of the eye allowing the setting of the fitting height of the eyewear to the user before the switching from the first lens holder to the second lens holder.

The method may further comprise moving S38' vertically the position of the frame headwear relative to the user to align a pupil center of an eye of the user with the landmarks allowing the setting of the fitting height of the eyewear to the user before the switching from the first lens holder to the second lens holder.

Such method and eyewear promote the detection of differences between the first lens and the second lens.

The method as disclosed hereinbefore has the advantage to provide a demonstration tool to support lens sales in-store allowing to switch together lenses while maintaining client's fittings parameters for another try.

Advantageously, such eyewear with a plurality of lens holders allows the client to efficiently experiment and compare benefits of different lenses: progressive addition lenses, bifocal lenses, trifocal lenses and single vision lenses, by quickly switching the corresponding lens holders while maintaining the fitting's parameters of the client in particular when the frame headwear is worn by the client.

The lenses may have different optical designs and/or different optical powers and/or different tint and/or different functional coatings allowing an easily comparison of their benefits by a client.

The invention has been described above with the aid of embodiments without limitation of the general inventive concept.

Furthermore, to increase visibility of the landmarks, said landmarks may be painted.

Many further modifications and variations will suggest themselves to those skilled in the art upon making reference to the foregoing illustrative embodiments, which are given by way of example only and which are not intended to limit the scope of the invention, that being determined solely by the appended claims.

In the claims, the word "comprising" does not exclude other elements or steps, and the indefinite article "a" or "an" does not exclude a plurality. The mere fact that different features are recited in mutually different dependent claims does not indicate that a combination of these features cannot be advantageously used. Any reference signs in the claims should not be construed as limiting the scope of the invention.

## Claims

1. An eyewear comprising:
- a frame headwear (12) configured to be worn by a user, the frame headwear comprising a front part (16), and
- at least one lens holder (14), each lens holder being configured to be removably fastened to the frame headwear in a predetermined mounted position and each lens holder comprising an associated pair of ophthalmic lenses (22, 24) attached to said lens holder, each lens of the associated pair of ophthalmic lenses (22, 24) being configured to be movable relative to the lens holder (14) while being attached to said lens holder.
wherein the eyewear comprises a set of at least two lens holders, each pair of ophthalmic lenses of the set comprises a right lens and a left lens,
wherein the set of at least one lens holder comprises:
- a first subset of at least one first lens holder, each first lens holder having an associated pair of single vision lenses with a first optical power, and
- a second subset of at least one second lens holder, each second lens holder having an associated pair of progressive or bifocal addition lenses with at least one zone having the first power.

2. The eyewear according to claim 1, wherein the lens holder comprises at least two landmarks, each of which locates a reference point of the ophthalmic lenses.

3. The eyewear according to claim 1 or 2, wherein at least one lens of the pair of ophthalmic lenses comprises a vertical graduated scale to evaluate the fitting height of the lens.

4. The eyewear according to any of the preceding claims 1 to 3 wherein at least one lens of the pair of ophthalmic lenses comprises a landmark to indicate the position of a near vision zone.

5. The eyewear according to any of the preceding claim 1 to 4, wherein each lens of the pair of ophthalmic lenses is movable relative to the associated lens holder along an axis corresponding to a nasal-temporal axis of the user when the eyewear is worn by the user such that the associated pair of ophthalmic lenses is positioned in front of the eyes of the user.

6. The eyewear according to any of the preceding claims 1 to 5, wherein the lens holder comprises a graduated scale which indicates the distance between the reference points of the two ophthalmic lenses of the lens holder.

7. The eyewear according to any of the preceding claims 1 to 6, wherein the each lens holder is magnetically fastened to the frame headwear.

8. The eyewear according to any of the preceding claims 1 to 7, wherein the lens holder comprises two circles jointed by a bridge, the lenses being positioned within the circles.

9. The eyewear according to any of the preceding claims 1 to 8, wherein a position of a nosepad of the frame headwear relative to the frame headwear is adjustable vertically when the eyewear is worn by the user and/or a position of the lens holder relative to the frame headwear is adjustable vertically when the eyewear is worn by the user such that the associated pair of ophthalmic lenses is positioned in front of the eyes of the user.

10. The eyewear according to any of the preceding claims 1 to 9, wherein the right lenses of the set having same right contour and optical centers of the right lenses being positioned in same place relative to the right contour and the left lenses of the set having same left contour and optical centers of the left lenses being positioned in same place relative to the left contour.

11. The eyewear according to any of the preceding claims 1 to 10, wherein each pair of ophthalmic lenses being different from one lens holder to another of the set, preferentially having different optical powers and/or different optical designs from one lens holder to another.

12. The eyewear according to any of the preceding claims 10 to 11, wherein the set of at least one lens holder comprises:
- a first subset comprising at least a first lens holder having an associated pair of single vision lenses having a first optical power, and a second lens holder having an associated pair of single vision lenses having a second optical power distinct from the first optical power, and
- a second subset comprising at least a third lens holder, the third lens holder having an associated pair of third lenses, the third lenses comprising at least a near vision zone having an optical power equal to the first optical power and a far vision zone having an optical power equal to the second optical power, the third lenses being progressive additional lenses or bifocal lenses or trifocal lenses.

13. Method for providing an eyewear to a user using an eyewear according to any of the preceding claims, the method comprising at least:
- providing the frame headwear;
- providing a lens holder;
- fastening the lens holder to the frame headwear;
- for each lens of the lens holder, moving the position of the lens relative to the lens holder to adjust the position of the lens to the pupillary distance of the user,
- providing another lens holder of the set;
- switching the lens holder with the other lens holder;
- for each lens of the other lens holder, moving the position of the lens relative to the other lens holder to adjust the position of the lens of the other lens holder to the pupillary distance of the user.

14. Method for providing an eyewear according to claims 13, each lens holder further comprising at least two landmarks each of which locates a reference point of an ophthalmic lens, the method further comprising using the landmarks to adjust the position of the lens to pupillary distance of the user.

15. Method for providing an eyewear to a user according to claim 13 or 14, wherein the method further comprises:
- moving vertically the position of the lens holder relative to the frame headwear or vertically a position of a nosepad of the frame headwear relative to the frame headwear to align a pupil center of an eye of the user with a landmark of a lens placed in front of the eye.

## Patentansprüche

1. Brille, umfassend:
- ein am Kopf tragbares Gestell (12), das konfiguriert ist, um von einem Benutzer getragen zu werden, wobei das am Kopf tragbare Gestell ein Vorderteil (16) umfasst, und
- mindestens einen Glashalter (14), wobei jeder Glashalter konfiguriert ist, um abnehmbar an dem am Kopf tragbaren Gestell in einer vorbestimmten montierten Position befestigt zu werden und wobei jeder Glashalter ein zugehöriges Paar Brillengläser (22, 24) umfasst, die an dem Glashalter befestigt sind, wobei jedes Glas von dem zugehörigen Paar Brillengläser (22, 24) so konfiguriert ist, dass es in Bezug auf den Glashalter (14) beweglich ist, während es an dem Glashalter befestigt ist,
wobei die Brille einen Satz von mindestens zwei Glashaltern umfasst, wobei jedes Paar Brillengläser des Satzes ein rechtes Glas und ein linkes Glas umfasst, wobei der Satz von mindestens einem Glashalter umfasst:
- einen ersten Teilsatz von mindestens einem ersten Glashalter, wobei jeder erste Glashalter ein zugehöriges Paar Einstärkengläser mit einer ersten Brechkraft aufweist, und
- einen zweiten Teilsatz aus mindestens einem zweiten Glashalter, wobei jeder zweite Glashalter ein zugehöriges Paar Gleitschicht- oder Bifokaladditionsgläser mit mindestens einer Zone aufweist, welche die erste Brechkraft aufweist.

2. Brille nach Anspruch 1, wobei der Glashalter mindestens zwei Orientierungspunkte umfasst, die jeweils einen Referenzpunkt der Brillengläser festlegen.

3. Brillenglas nach Anspruch 1 oder 2, wobei mindestens ein Glas des Paars Brillengläser eine vertikale Teilstrichskala umfasst, um die Einschleifhöhe des Glases zu ermitteln.

4. Brille nach einem der vorhergehenden Ansprüche 1 bis 3, wobei mindestens ein Glas des Paars Brillengläser einen Orientierungspunkt umfasst, um die Position einer Nahsichtzone anzuzeigen.

5. Brille nach einem der vorhergehenden Ansprüche 1 bis 4, wobei jedes Glas des Paars Brillengläser in Bezug auf den zugehörigen Glashalter entlang einer Achse beweglich ist, die einer nasal-temporalen Achse des Benutzers entspricht, wenn die Brille von dem Benutzer getragen wird, sodass das zugehörige Paar Brillengläser vor den Augen des Benutzers positioniert wird.

6. Brille nach einem der vorhergehenden Ansprüche 1 bis 5, wobei der Glashalter eine Teilstrichskala umfasst, die den Abstand zwischen den Referenzpunkten der zwei Brillengläser des Glashalters anzeigt.

7. Brille nach einem der vorhergehenden Ansprüche 1 bis 6, wobei jeder Glashalter magnetisch an dem am Kopf tragbaren Gestell befestigt ist.

8. Brille nach einem der vorhergehenden Ansprüche 1 bis 7, wobei der Glashalter zwei Kreise umfasst, die durch eine Brücke verbunden sind, wobei die Gläser in den Kreisen positioniert sind.

9. Brille nach einem der vorhergehenden Ansprüche 1 bis 8, wobei eine Position eines Nasenpolsters des am Kopf tragbaren Gestells in Bezug auf das am Kopf tragbaren Gestell vertikal einstellbar ist, wenn die Brille von dem Benutzer getragen wird, und/oder wobei eine Position des Glashalters in Bezug auf das am Kopf tragbaren Gestell vertikal einstellbar ist, wenn die Brille von dem Benutzer getragen wird, sodass das zugehörige Paar Brillengläser vor den Augen des Benutzers positioniert wird.

10. Brille nach einem der vorhergehenden Ansprüche 1 bis 9, wobei die rechten Gläser des Satzes, die eine gleiche rechte Kontur und gleiche optische Zentren der rechten Gläser aufweisen, an dem gleichen Ort in Bezug auf die rechte Kontur positioniert werden und wobei die linken Gläser des Satzes, die eine gleiche linke Kontur und gleiche optische Zentren der linken Gläser aufweisen, an dem gleichen Ort in Bezug auf die linke Kontur positioniert werden.

11. Brille nach einem der vorhergehenden Ansprüche 1 bis 10, wobei jedes Paar Brillengläser von einem Glashalter zu einem anderen Glashalter des Satzes unterschiedlich sind, wobei sie vorzugsweise unterschiedliche Brechkräfte und/oder unterschiedliche optische Auslegungen von einem Glashalter zu einem anderen Glashalter aufweisen.

12. Brille nach einem der vorhergehenden Ansprüche 10 bis 11, wobei der Satz von mindestens einem Glashalter umfasst:
- einen ersten Teilsatz, der mindestens einen ersten Glashalter, der ein zugehöriges Paar Einstärkengläser aufweist, die eine erste Brechkraft aufweisen, und einen zweiten Glashalter umfasst, der ein zugehöriges Paar Einstärkengläser aufweist, die eine zweite Brechkraft aufweisen, die verschieden von der ersten Brechkraft ist, und
- einen zweiten Teilsatz, der mindestens einen dritten Glashalter umfasst, wobei der dritte Glashalter ein zugehöriges Paar dritter Gläser aufweist, wobei die dritten Gläser mindestens eine Nahsichtzone, die eine Brechkraft gleich der ersten Brechkraft aufweist, und eine Fernsichtszone umfassen, die eine Brechkraft gleich der zweiten Brechkraft aufweist, wobei die dritten Gläser Gleitsichtadditionsgläser oder Bifokalgläser oder Trifokalgläser sind.

13. Verfahren zum Bereitstellen einer Brille für einen Benutzer, der eine Brille nach einem der vorhergehenden Ansprüche verwendet, wobei das Verfahren mindestens umfasst:
- Bereitstellen des am Kopf tragbaren Gestells;
- Bereitstellen eines Glashalters;
- Befestigen des Glashalters an dem am Kopf tragbaren Gestell;
- für jedes Glas des Glashalters, Bewegen der Position des Glases in Bezug auf den Glashalter, um die Position des Glases des Glases auf die Pupillendistanz des Benutzers einzustellen,
- Bereitstellen eines weiteren Glashalters des Satzes;
- Ersetzen des Glashalters durch den weiteren Glashalter;
- für jedes Glas des weiteren Glashalters, Bewegen der Position des Glases in Bezug auf den weiteren Glashalter, um die Position des Glases des weiteren Glashalters auf die Pupillendistanz des Benutzers einzustellen.

14. Verfahren zum Bereitstellen einer Brille nach Anspruch 13, wobei jeder Glashalter ferner mindestens zwei Orientierungspunkte umfasst, die jeweils einen Referenzpunkt eines Brillenglases festlegen, wobei das Verfahren ferner ein Verwenden der Orientierungspunkte umfasst, um die Position des Glases auf die Pupillendistanz des Benutzers einzustellen.

15. Verfahren zum Bereitstellen einer Brille für einen Benutzer nach Anspruch 13 oder 14, wobei das Verfahren ferner umfasst:
- vertikales Bewegen der Position des Glashalters in Bezug auf das am Kopf tragbare Gestell oder vertikales Bewegen einer Position eines Nasenpolsters des am Kopf tragbaren Gestells in Bezug auf das am Kopf tragbare Gestell, um ein Pupillenzentrum eines Auges des Benutzers auf einen Orientierungspunkt eines Glases auszurichten, das vor dem Auge des Benutzers angeordnet ist.

## Revendications

1. Lunettes comprenant :
- une monture de coiffe (12) configuré pour être porté par un utilisateur, la monture de coiffe comprenant une partie avant (16), et
- au moins un support de verre (14), chaque support de verre étant configuré pour être fixé de manière amovible à la monture de coiffe dans une position de montage prédéterminée et chaque support de verre comprenant une paire associée de verres ophtalmiques (22, 24) fixée audit support de verre, chaque verre de la paire associée de verres ophtalmiques (22, 24) étant configuré pour être mobile par rapport au support de verre (14) tout en étant fixé audit support de verre, lorsque les lunettes comprennent un ensemble d'au moins deux supports de verre, chaque paire de verres ophtalmiques de l'ensemble comprenant un verre droit et un verre gauche,
l'ensemble d'au moins un support de verre comprenant :
- un premier sous-ensemble d'au moins un premier support de verre, chaque premier support de verre ayant une paire associée de verres unifocaux ayant une première puissance optique, et
- un deuxième sous-ensemble d'au moins un deuxième support de verre, chaque deuxième support de verre ayant une paire associée de verres d'addition progressifs ou bifocaux avec au moins une zone ayant la première puissance.

2. Lunettes selon la revendication 1, le support de verre comprenant au moins deux repères, chacun d'eux localisant un point de référence des verres ophtalmiques.

3. Lunettes selon la revendication 1 ou 2, au moins un verre de la paire de verres ophtalmiques comprenant une échelle graduée verticale pour évaluer la hauteur d'ajustement du verre.

4. Lunettes selon l'une quelconque des revendications précédentes 1 à 3, au moins un verre de la paire de verres ophtalmiques comprenant un repère pour indiquer la position d'une zone de vision de près.

5. Lunettes selon l'une quelconque des revendications précédentes 1 à 4, chaque verre de la paire de verres ophtalmiques étant mobile par rapport au support de verre associé le long d'un axe correspondant à un axe naso-temporal de l'utilisateur lorsque les lunettes sont portées par l'utilisateur de sorte que la paire de verres ophtalmiques associée est positionnée devant les yeux de l'utilisateur.

6. Lunettes selon l'une quelconque des revendications précédentes 1 à 5, le support de verre comprenant une échelle graduée qui indique la distance entre les points de référence des deux verres ophtalmiques du support de verre.

7. Lunettes selon l'une quelconque des revendications précédentes 1 à 6, chaque support de verre étant fixé magnétiquement à la monture de coiffe.

8. Lunettes selon l'une quelconque des revendications précédentes 1 à 7, le support de verre comprenant deux cercles reliés par un pont, les verres étant positionnés à l'intérieur des cercles.

9. Lunettes selon l'une quelconque des revendications précédentes 1 à 8, une position d'un couvre-nez de la monture de coiffe par rapport à la monture de coiffe étant réglable verticalement lorsque la monture de lunettes est portée par l'utilisateur et/ou une position du support de verre par rapport à la monture de coiffe étant réglable verticalement lorsque la monture de lunettes est portée par l'utilisateur de sorte que la paire associée de verres ophtalmiques est positionnée devant les yeux de l'utilisateur.

10. Lunettes selon l'une quelconque des revendications précédentes 1 à 9, les verres droits de l'ensemble ayant le même contour droit et les centres optiques des verres droits étant positionnés au même endroit par rapport au contour droit et les verres gauches de l'ensemble ayant le même contour gauche et les centres optiques des verres gauches étant positionnés au même endroit par rapport au contour gauche.

11. Lunettes selon l'une quelconque des revendications précédentes 1 à 10, chaque paire de verres ophtalmiques étant différente d'un support de verre à l'autre de l'ensemble, ayant de préférence des puissances optiques différentes et/ou des conceptions optiques différentes d'un support de verre à l'autre.

12. Lunettes selon l'une quelconque des revendications précédentes 10 et 11, l'ensemble d'au moins un support de verre comprenant :
- un premier sous-ensemble comprenant au moins un premier support de verre ayant une paire associée de verres unifocaux ayant une première puissance optique, et un deuxième support de verre ayant une paire associée de verres unifocaux ayant une deuxième puissance optique distincte de la première puissance optique, et
- un deuxième sous-ensemble comprenant au moins un troisième support de verre, le troisième support de verre ayant une paire associée de troisièmes verres, les troisièmes verres comprenant au moins une zone de vision de près ayant une puissance optique égale à la première puissance optique et une zone de vision de loin ayant une puissance optique égale à la deuxième puissance optique, les troisièmes verres étant des verres d'addition progressifs ou des verres bifocaux ou des verres trifocaux.

13. Procédé pour fournir des lunettes à un utilisateur en utilisant des lunettes selon l'une quelconque des revendications précédentes, le procédé comprenant au moins :
- la fourniture de la monture de coiffe ;
- la fourniture d'un support de verre ;
- la fixation du support de verre à la monture de coiffe ;
- pour chaque verre du support de verre, le déplacement de la position du verre par rapport au support de verre pour ajuster la position du verre à la distance pupillaire de l'utilisateur,
- la fourniture d'un autre support de verre de l'ensemble ;
- le passage du support de verre à l'autre support de verre ;
- pour chaque verre de l'autre support de verre, le déplacement de la position du verre par rapport à l'autre support de verre pour ajuster la position du verre de l'autre support de verre à la distance pupillaire de l'utilisateur.

14. Procédé pour fournir des lunettes selon la revendication 13, chaque support de verre comprenant en outre au moins deux repères dont chacun localise un point de référence d'un verre ophtalmique, le procédé comprenant en outre l'utilisation des repères pour ajuster la position du verre à la distance pupillaire de l'utilisateur.

15. Procédé pour fournir des lunettes à un utilisateur selon la revendication 13 ou 14, le procédé comprenant en outre :
- le déplacement vertical de la position du support de verre par rapport à la monture de coiffe ou verticalement de la position d'un couvre-nez de la monture de coiffe par rapport à la monture de coiffe pour aligner le centre de la pupille d'un œil de l'utilisateur avec un repère d'un verre placé devant l'œil.
